# EUROPÄISCHE PATENTSCHRIFT

(11) **EP 1 594 864 B1**
(45) Veröffentlichungstag und Bekanntmachung des Hinweises auf die Patenterteilung: **22.10.2008**
(21) Anmeldenummer: 04703167.9
(22) Anmeldetag: 19.01.2004
(51) Int. Cl.: C07D 405/12, A61K 31/404, A61P 25/22, A61P 25/24

(54) **BENZOFURANOXYETHYLAMINE ALS ANTIDEPRESSIVA UND ANXIOLYTIKA**
BENZOFURAN OXYETHYLAMINES SERVING AS ANTIDEPRESSANT DRUGS AND ANXIOLYTIC DRUGS
BENZOFURANOXYETHYLAMINES SERVANT D'ANTIDEPRESSEURS ET D'ANXIOLYTIQUES

(30) Priorität: 18.02.2003 DE 10306941
(43) Veröffentlichungstag der Anmeldung: 16.11.2005
(73) Patentinhaber: Merck Patent GmbH, 64293 Darmstadt (DE)
(72) Erfinder: HÖLZEMANN, Günter, 64342 Seeheim-Jugenheim (DE); SCHIEMANN, Kai, 64342 Seeheim-Jugenheim (DE); BÖTTCHER, Henning, 64287 Darmstadt (DE); HEINRICH, Timo, 64823 Gross-Umstadt (DE); SEYFRIED, Christoph, 64342 Seeheim-Jugenheim (DE); LEIBROCK, Joachim, 64319 Pfungstadt (DE); VAN AMSTERDAM, Christoph, 64295 Darmstadt (DE); BARTOSZYK, Gerd, 64331 Weiterstadt (DE)
(86) Internationale Anmeldenummer: PCT/EP2004/000348
(87) Internationale Veröffentlichungsnummer: WO 2004/074281

(56) Entgegenhaltungen:
- EP-A- 0 648 767
- WO-A-99/51575
- WO-A-99/51576
- WO-A-02/083666
- US-A- 4 404 217
- US-A- 5 436 264

## Beschreibung

Die Erfindung betrifft Benzofuranoxyethylamine der Formel I 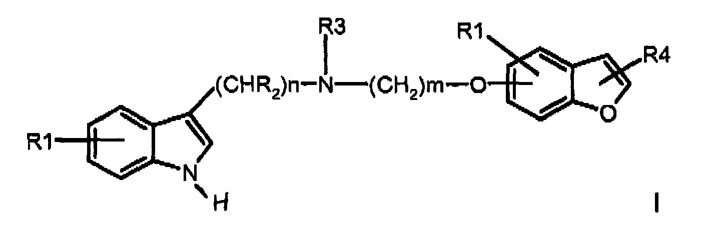 worin
- R¹: H, Ein- oder Zweifachsubstitution durch CN,
- R: OH, OA, NH₂, NHA oder NA₂
- R²,R³: H oder A
- R⁴: Ein- oder Zweifachsubstitution durch COR
- A: Alkyl mit 1, 2, 3, 4, 5 oder 6 Atomen
- Hal: F, Cl, Br oder I
- m: 2, 3, 4, 5 oder 6
- n: 1, 2, 3 oder 4
bedeuten,
sowie ihre Solvate und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden.

Andere Indolderivate kennt man aus EP 648767 (Merck) oder aus WO 99/51575 (American Home Prod.).

Es wurde gefunden, dass die erfindungsgemäßen Verbindungen der Formel I und ihre physiologisch unbedenklichen Säureadditionssalze bei guter Verträglichkeit wertvolle pharmakologische Eigenschaften besitzen, da sie Wirkungen auf das Zentralnervensystem, insbesondere 5-HT-reuptake hemmende Wirkungen, aufweisen, indem sie die serotoninerge Transmission beeinflussen. Sie weisen eine Affinität zu den 5-HTₓ- Rezeptoren auf, wobei der Subtyp 5 HT_{1A} besonders bevorzugt ist. Bei 5 HTₓ bedeutet 1A, 1 D, 2A, 2C, 3 oder 4.
Da die Verbindungen auch die Serotonin-Wiederaufnahme hemmen, eignen sie sich insbesondere als Antidepressiva und Anxiolytika. Die Verbindungen zeigen serotonin-agonistische und -antagonistische Eigenschaften. Sie hemmen die Bindung von tritiierten Serotoninliganden an hippocampale Rezeptoren (Cossery et al., European J. Pharmacol. 140 (1987), 143-155) und hemmen die synaptosomale Serotoninwiederaufnahme (Sherman et al., Life Sci. 23 (1978), 1863-1870).
Zum ex-vivo Nachweis der Serotoninwiederaufnahmehemmung wird die synaptosomale Aufnahmehemmung (Wong et al., Neuropsychopharmacol. 8 (1993), 23-33) und der p-Chloramphetaminantagonismus (Fuller et al., J. Pharmacol. Exp.Ther. 212 (1980), 115-119) herangezogen.

Die Bindungseigenschaften der Verbindungen der Formel I lassen sich u.a. durch bekannte 5-HT_{1A}-(Serotonin)-Bindungstests bestimmen (5-HT_{1A}-(Serotonin)-Bindungstest: Matzen et al., J. Med. Chem., 43, 1149-1157, (2000) insbesondere Seite 1156 mit Verweis auf Eur. J. Pharmacol.: 140, 143-155 (1987).

Die erfindungsgemäßen Verbindungen können zur Behandlung von Krankheiten eingesetzt werden, die mit dem Serotonin- Neurotransmittersystem verbunden sind und bei denen z.B. hochaffine Serotoninrezeptoren (5-HT_{1A}-Rezeptoren) beteiligt sind.

Die Verbindungen der Formel I eignen sich daher sowohl in der Veterinär- als auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Insbesondere sind sie jedoch geeignet als Arzneimittelwirkstoffe für Anxiolytika, Antidepressiva, Antipsychotika, Neuroleptika. Antihypertonika und/oder zur positiven Beeinflussung von Zwangsverhalten (obsessive-compulsive disorder, OCD), Angstzuständen, Panikattacken, Psychosen, Anorexie, wahnhaften Zwangsvorstellungen, Migräne, der Alzheimer Krankheit, Schlafstörungen, tardiver Dyskinesien, Lernstörungen, altersabhängiger Erinnerungsstörungen, Essstörungen wie Bulimie, Drogenmissbrauch und/oder Sexualfunktionsstörungen.

Eine wichtige Indikation für die Verabreichung der Verbindung der allgemeinen Formel I sind Psychosen jeder Art, insbesondere auch Geisteskrankheiten aus dem Formenkreis der Schizophrenie. Darüber hinaus können die Verbindungen auch zur Verminderung von kognitiven Leistungsstörungen, also zur Verbesserung der Lernfähigkeit und des Gedächtnisses, eingesetzt werden. Auch zur Bekämpfung der Symptome von Morbus-Alzheimer sind die Verbindungen der allgemeinen Formel I geeignet. Darüber hinaus eignen sich die erfindungsgemäßen Substanzen der allgemeinen Formel I zur Prophylaxe und Kontrolle von Hirninfarkten (Apoplexia cerebri), wie Hirnschlag und zerebrale Ischemie. Ferner kommen die Substanzen zur Behandlung von Krankheiten wie krankhafte Angstzustände, Übererregung, Hyperaktivität und Ausmerksamkeitsstörungen bei Kindern und Jugendlichen, tiefgreifende Entwicklungsstörungen und Störungen des Sozialverhaltens mit geistiger Retardierung, Depression, Zwangserkrankungen im engeren (OCD) und weiteren Sinne (OCSD) bestimmte sexuelle Funktionsstörungen, Schlafstörungen und Störungen in der Nahrungsaufnahme, sowie solcher Psychiatrischer Symptome in Rahmen der Altersdemenz und der Demenz vom Alzheimer-Typ, also Krankheiten des zentralen Nervensystems im weitesten Sinn, in Frage.

Die Verbindungen der Formel I eignen sich ebenfalls zur Behandlung von extrapyramidalen Bewegungserkrankungen, zur Behandlung von Nebenwirkungen, die bei der Behandlung von extrapyramidalen Bewegungserkrankungen mit konventionellen Anti-Parkinson-Medikamenten auftreten oder zur Behandlung von extrapyramidalen Symptomen (EPS), die durch Neuroleptika induziert werden.

Extrapyramidale Bewegungserkrankungen sind beispielsweise idiopathische Parkinson'sche Krankheit, Parkinson-Syndrom, dyskinetische choreatische oder dystonische Syndrome, Tremor, Gilles de la Torette Syndrom, Ballismus, Muskelkrampf, Restless Legs Syndrom, Wilsons-Krankheit, Lewy bodies Dementia, Huntington und Tourette Syndrom.

Die erfindungsgemäßen Verbindungen eignen sich insbesondere auch zur Behandlung von neurodegenerativen Erkrankungen, wie z.B. Lathyrismus, Alzheimer, Parkinson, und Huntington.

Die Verbindungen der Formel I eignen sich insbesondere zur Behandlung von Nebenwirkungen, die bei der Behandlung der idiopathischen Parkinson-Krankheit mit konventionellen Parkinson-Medikamenten auftreten. Sie können deshalb auch als Zusatztherapie (add-on therapy) bei der Behandlung der Parkinson-Krankheit verwendet werden.

Bekannte Parkinson-Medikamente sind Arzneimittel wie L-Dopa (Levodopa) und L-Dopa kombiniert mit Benserazid oder Carbidopa, Dopamin Agonisten wie Bromocriptin, Apomorphin, Cabergolin, Pramipexol, Ropinirol, Pergolid, Dihydro-α-ergocriptin oder Lisurid und alle Medikamente, die eine Stimulierung des Dopamin-Rezeptors bewirken, Inhibitoren der Catechol-O-methyl-Transferase (COMT) wie Entacapon oder Tolcapon, Inhibitoren der Monoamin Oxidase (MAO) wie Selegilin und Antagonisten von N-Methyl-D-aspartat (NMDA) Rezeptoren wie Amantadin oder Budipin.

Die Verbindungen der allgemeinen Formel I und ihre verträglichen Salze und Solvate können somit als aktive Inhaltsstoffe für Arzneimittel wie Anxiolytika, Antidepressiva, Neuroleptika und/oder Antihypertonika eingesetzt werden.

Ein Maß für die Aufnahme eines Arzneimittelwirkstoffs in einen Organismus ist seine Bioverfügbarkeit.
Wird der Arzneimittelwirkstoff in Form einer Injektionslösung dem Organismus intravenös zugefügt, so liegt seine absolute Bioverfügbarkeit, d.h. der Anteil des Pharmakons, der unverändert im systemischen Blut, d.h. in den großen Kreislauf gelangt, bei 100%.
Bei oraler Vergabe eines therapeutischen Wirkstoffs liegt der Wirkstoff in der Regel als Feststoff in der Formulierung vor und muß sich daher zuerst auflösen, damit er die Eintrittsbarrieren, beispielsweise den Gastrointestinaltrakt, die Mundschleimhaut, nasale Membranen oder die Haut, insbesondere das Stratum corneum, überwinden kann bzw. vom Körper resorbiert werden kann. Daten zur Pharmakokinetik, d.h. zur Bioverfügbarkeit können analog zu der Methode von J. Shaffer et al, J. Pharm. Sciences, 1999, 88, 313-318 erhalten werden.

Ein weiteres Maß für die Resorbierbarkeit eines therapeutischen Wirkstoffes ist der logD-Wert, denn dieser Wert ist ein Maß für die Lipophilie eines Moleküls.

Soweit die Verbindungen der allgemeinen Formel I optisch aktiv sind, umfasst die Formel I sowohl jede isolierte optische Antipode als auch die entsprechenden gegebenenfalls racemischen Gemische in jeder denkbaren Zusammensetzung.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Additionsverbindungen mit Alkoholen, wie z.B. mit Methanol oder Ethanol.

Gegenstand der Erfindung sind die Verbindungen der Formel I und ihre Salze und Solvate nach Anspruch 1 sowie ein Verfahren zur Herstellung von Verbindungen der Formel I sowie ihrer Salze, Solvate und Stereoisomere, dadurch gekennzeichnet, dass man
eine Verbindung der Formel II 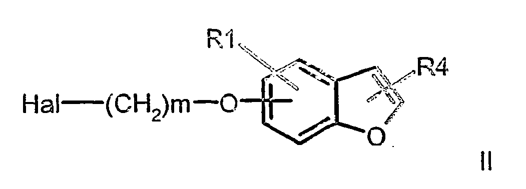 worin
R¹, R⁴, Hal und m die in Anspruch 1 angegebenen Bedeutungen haben mit einer Verbindung der Formel III 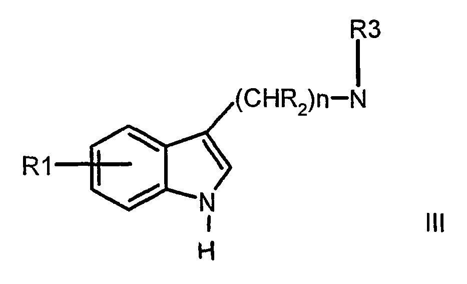 worin
R¹, R², R³ und n
die in Anspruch 1 angegebenen Bedeutungen haben,
umsetzt und/oder
eine basische oder saure Verbindung der Formel I durch Behandeln mit einer Säure oder Base in eines ihrer Salze oder Solvate umwandelt.
Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z.B. in Int. J. Pharm. 115, 61-67 (1995) beschrieben ist.

A bedeutet Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, und ist unverzweigt (linear) oder verzweigt. Besonders bevorzugt ist Methyl oder Ethyl.

Hal bedeutet vorzugsweise F, Cl, Br aber auch I.

OA bedeutet vorzugsweise Methoxy, Ethoxy, Propoxy, Isopropoxy, Butoxy oder tert.-Butoxy.

R bedeutet vorzugsweise NH₂, OH, O-Methyl oder O-Ethyl.
R¹ bedeutet vorzugsweise CN.
R² bedeutet vorzugsweise H oder Methyl.
R³ bedeutet vorzugsweise H oder Methyl. R⁴ bedeutet vorzugsweise CONH₂, COOC₂H₅, CONHA
m bedeutet vorzugsweise 2 oder 3.
n bedeutet vorzugsweise 2 oder 4.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Die Verbindungen der Formel I nach Anspruch 1 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so dass man sie aus dem Reaktionsgemisch nicht isoliert, sondern sofort weiter zu den Verbindungen der Formel I nach Anspruch 1 umsetzt.

Die Ausgangsverbindungen der Formel II und III sind in der Regel bekannt. Sind sie neu, so können sie aber nach an sich bekannten Methoden hergestellt werden.

Verbindungen der Formel I können durch Umsetzung der Verbindungen der Formel II mit Verbindungen der Formel III unter Standardbedingungen hergestellt werden.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Ethyldiisopropylamin, Triethylamin, Dimethylanilin, Pyridin oder Chinolin kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, N-Methylpyrrolidon, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, schweflige Säure, Dithionsäure, Salpetersäure, Halogenwasserstoffsäuren wie Chlorwasserstoffsäure oder Bromwasser-stoffsäure, Phosphorsäuren wie z.B. Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Benzolsulfonsäure, Trimethoxybenzoesäure, Adamantancarbonsäure, p-Toluolsulfonsäure, Glycolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glucose-1-phosphat, Naphthalin-mono- und disulfonsäuren oder Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall- oder in die entsprechenden Ammoniumsalze umgewandelt werden.

Gegenstand der Erfindung sind auch die Verbindungen der Formel I nach Anspruch 1 und ihre physiologisch unbedenklichen Salze oder Solvate als Arzneimittelwirkstoffe.

Weiterhin sind Gegenstand der Erfindung Verbindungen der Formel I und ihre physiologisch unbedenklichen Salze oder Solvate als 5HT_{1A}-, 5HT_{1D}, 5HT_{2A}- Agonisten und als Inhibitoren der 5HT-Wiederaufnahme.

Gegenstand der Erfindung sind auch die Verbindungen der Formel I nach Anspruch 1 und ihre physiologisch unbedenklichen Salze oder Solvate zur Anwendung bei der Bekämpfung von Krankheiten.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre Solvate und Stereoisomere, einschließlich deren Mischungen in allen Verhältnissen, sowie gegebenenfalls Träger- und/oder Hilfsstoffe.

Ein weiterer Gegenstand der Erfindung ist die Verwendung einer Verbindung der allgemeinen Formel I und/oder Solvate und Stereoisomere, einschließlich deren Mischungen in allen Verhältnissen zur Herstellung eines Arzneimittels, welches sich zur Behandlung von menschlichen oder tierischen Krankheiten, insbesondere von Krankheiten des zentralen Nervensystems wie krankhafte Spannungszustände, Depression und/oder Psychosen, zur Verminderung von Nebenwirkungen bei der Behandlung von Bluthochdruck (z.B. mit alpha- Methyldopa), zur Behandlung von endokrinologischen und/oder gynäkologischen Krankheiten, z.B. zur Behandlung von Agromegalie, Hypogonadismus, sekundärer Amenorrhoe, dem postmenstruellen Syndrom und unerwünschter Laktation in der Pubertät und zur Prophylaxe und Therapie von zerebralen Krankheiten (z.B. von Migräne) insbesondere in der Gereatrle in ähnlicher Weise wie bestimmte Ergot-Alkaloide und zur Kontrolle und Prophylaxe von Himinfarkt (Apoplexia cerebri) wie Hirnschlag und zerebraler Ischaemie, zur Behandlung von extrapyramidalen Bewegungserkrankungen, zur Behandlung von Nebenwirkungen, die bei der Behandlung von extrapyramidalen Bewegungserkrankungen mit konventionellen Anti-Parkinson-Medikamenten auftreten oder zur Behandlung von extrapyramidalen Symptomen (EPS), die durch Neuroleptika induziert werden, eignet. Darüber hinaus sind die pharmazeutischen Zubereitungen und Arzneimittel, die eine Verbindung der allgemeinen Formel I enthalten, zur Verbesserung des kognitiven Leistungsvermögens und zur Behandlung von Morbus-Alzheimer-Symptomen geeignet.

Insbesondere eignen sich solche Arzneimittel zur Behandlung von Geisteskrankheiten aus dem Formenkreis der Schizophrenie und zur Bekämpfung von psychotischen Angstzuständen. Der Begriff Behandlung schließt im Rahmen der Erfindung Prophylaxe und Therapie menschlicher oder tierischer Krankheiten ein.
Gegenstand der Erfindung ist weiterhin die Verwendung von Verbindungen der Formel I und/oder ihrer Solvate und Stereoisomere, einschließlich deren Mischungen in allen Verhältnissen, zur Herstellung eines Arzneimittels zur Bekämpfung von Krankheiten, die mit dem Serotonin-Neurotransmittersystem verbunden sind und bei denen hochaffine Serotoninrezeptoren (5-HT_{1A}-Rezeptoren) und/oder 5HT_{1D}-Rezeptoren beteiligt sind.

Gegenstand der Erfindung ist weiterhin die Verwendung von Verbindungen der Formel I und/oder ihrer Solvate und Stereoisomere, einschließlich deren Mischungen in allen Verhältnissen, zur Herstellung eines Arzneimittels als Anxiolytikum, Antidepressivum, Neuroleptikum und/oder Antihypertensivum.

Die Substanzen der allgemeinen Formel I werden normalerweise analog zu bekannten, kommerziell erhältlichen pharmazeutischen Zubereitungen (z.B. von Bromocriptin und Dihydroergocornin), vorzugsweise in Dosen von zwischen 0,2 und 500 mg, insbesondere von zwischen 0,2 und 15 mg pro Dosiseinheit verabreicht. Die tägliche Dosiseinheit ist zwischen 0,001 und 10 mg pro kg Körpergewicht. Geringe Dosen (von zwischen 0,2 und 1 mg pro Dosiseinheit, 0,001 bis 0,005 mg pro kg Körpergewicht) sind besonders geeignet für pharmazeutische Zubereitungen zur Behandlung von Migräne. Eine Dosis zwischen 10 und 50 mg pro Dosiseinheit wird für andere Indikationen bevorzugt. Allerdings hängt die zu verabreichende Dosis von einer Vielzahl von Faktoren ab, z.B. von der Wirksamkeit der entsprechenden Komponente, dem Alter, dem Körpergewicht und der allgemeinen Verfassung des Patienten.

Gegenstand der Erfindung sind ferner Arzneimittel enthaltend mindestens eine Verbindung der Formel I und/oder ihre Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel, durch präparative HPLC und/oder durch Kristallisation. Die gereinigten Verbindungen werden gegebenenfalls gefriergetrocknet.
- Massenspektrometrie (MS):: EI (Elektronenstoß-Ionisation) M⁺
FAB (Fast Atom Bombardment) (M+H)⁺
ESI (Electrospray Ionization) (M+H)⁺ (wenn nichts anderes angegeben)

### Beispiel 1 (nur zur technischen Information)

### Darstellung von 7-{2-[2-(5-Fluoro-1H-indol-3-yl)-ethylamino]-ethoxy}-benzofuran-2-carboxylic acid amide:

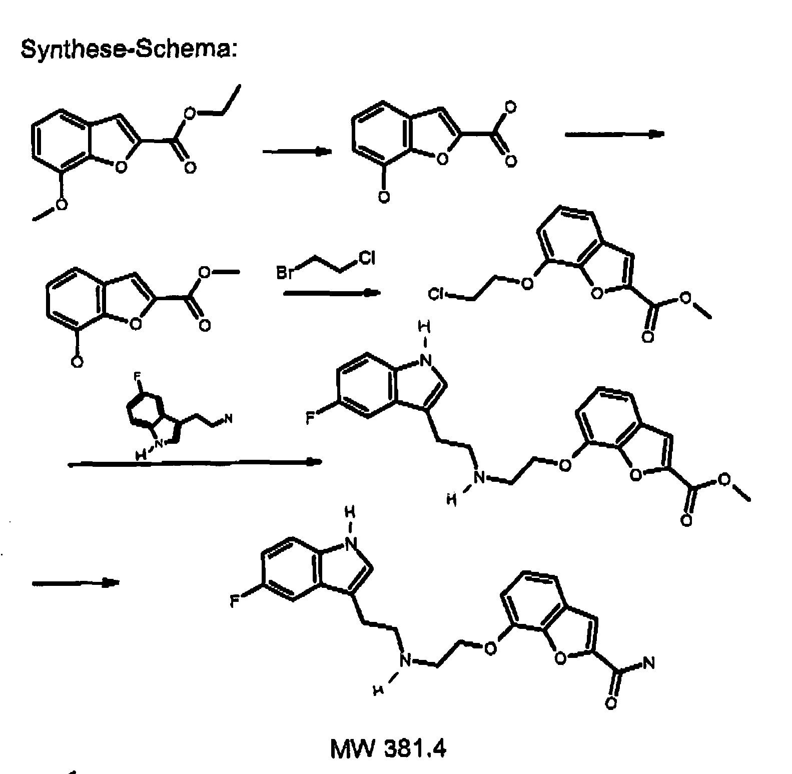

### Herstellung von 7-Hydroxy-benzofuran-2-carboxylic acid ethyl ester:

2 g 7-Methoxy-benzofuran-2-carboxylic acid ethyl ester werden in 50 ml Dichloromethan gelöst. Die Lösung wird auf -15°C abgekühlt und unter N₂ werden 8.5 ml Bortribromid dazugegeben. Die Reaktionsmischung wird ca. 3 h bei dieser Temperatur und über Nacht bei Raumtemperatur gerührt.
Es werden 10 ml Methanol und 20 ml NaCl-Lösung dazugegeben und mit Ethylacetat extrahiert.
Die organischen Phasen werden vereinigt, getrocknet und eingeengt. Der Rückstand wird mit Petrolether verrieben, abfiltriert und getrocknet. Man erhält 1.87 g 7-Hydroxy-benzofuran-2-carboxylic acid.
Die Carbonsäure wird in 30 ml Ethanol gelöst und auf ca. 0° C abgekühlt. Man tropft 1.5 ml Thionylchlorid langsam zu und lässt über Nacht bei Raumtemperatur rühren.
Die Lösung wird mit Wasser versetzt, wobei das gewünschte Produkt ausfällt. Man erhält 1.5 g des 7-Hydroxy-benzofuran-2-carboxylic acid ethyl esters.
EI-MS (M)⁺ 205

In gleicher Weise wird der 7-Hydroxy-benzofuran-2-carboxylic acid methyl ester erhalten, wenn anstatt Ethanol Methanol verwendet wird.
EI-MS (M)⁺ 192

### Herstellung von 7-(2-Chloro-ethoxy)-benzofuran-2-carboxylic acid methyl ester:

2.8 g des vorher erhaltenen 7-Hydroxy-benzofuran-2-carboxylic acid methyl esters und 5.2 ml 1-Brom-2-chlorethan werden in 60 ml Aceton gelöst und es werden 2 g Kaliumcarbonat und 20 mg Kaliumjodid zugegeben. Das Ganze wird zwei Tage unter Rückfluss gekocht.
Zur Aufarbeitung wird das Lösungsmittel am Rotavapor abgezogen. Der Rückstand wird mit Wasser versetzt und dann mit Essigester extrahiert. Die gesamten organischen Phasen werden mit wasserfreiem Natriumsulfat getrocknet, filtriert und das Lösungsmittel wird am Rotavapor abgezogen. Man erhält 2.8 g der gewünschten Substanz.

HPLC-MS (M+H)⁺ 255

### Herstellung von 7-{2-[2-(5-Fluoro-1H-indol-3-yl)-ethylamino]-ethoxy}-benzofuran-2-carboxylic acid amide:

318 mg des oben hergestellten Materials und 215 mg 2-(5-Fluoro-1H-indol-3-yl)-ethylamine werden in 10 ml Acetonitril gelöst. Es werden 415 mg Kaliumcarbonat und 83 mg Kaliumjodid zugegeben. Die Mischung lässt man ca. 3 Tage unter Rückfluss kochen.

Zur Aufarbeitung wird das Reaktionsgemisch in Wasser/Eis gegeben. Es erfolgt eine Extraktion mit Essigester. Die gesamten organischen Phasen werden mit wasserfreiem Natriumsulfat getrocknet, filtriert und das Lösungsmittel wird am Rotavapor abgezogen.
Zur Aufreinigung wird eine Flash-Kieselgelchromatographie durchgeführt (Dichloromethan/Methanol 9/1).

80 mg von 7-{2-[2-(5-Fluoro-1H-indol-3-yl)-ethylamino]-ethoxy}-benzofuran-2-carboxylic acid ethyl ester werden isoliert.
HPLC-ESI-MS (M + H)⁺ 397

Der Ester wird in 2 ml Methanol gelöst und mit 4 ml Ammoniaklösung 25% versetzt. Das Ganze rührt über Nacht bei Raumtemperatur.
Zur Aufreinigung wird eine präparative HPLC durchgeführt:
Säule: RP 18 (7mum) Lichrosorb 250 x 25 (Art.Nr. 151494)

- Fließmittel:: A = 98 H₂O, 2 CH₃CN + 0.1% TFA
B = 10 H₂O, 90 CH₃CN + 0.1% TFA

UV: 225 NM;
Fluss: 10ml/min (1 Fraktion = 1Minute)

| | | |
|---|---|---|
| Gradient: | 0 min | 15% B |
| | 5 min | 15% B |
| | 50 min | 80% B |
| | 70 min | 95% |

Man erhält 23 mg der gewünschten Substanz.
HPLC-ESI-MS (M+H)⁺ 382

b) Alle folgenden Produkte werden nach dem oben angegebenen Syntheseschema und den Arbeitsvorschriften analog der Beispielsynthese erhalten.

| **BEISPIELE** | **MW** | | **SALZ** | **MW-SALZ** | **NAME** |
|---|---|---|---|---|---|
| 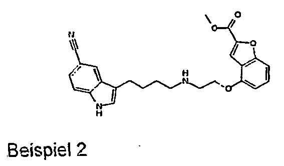 | 431,5 | | trifluoroacetate | 545.5 | 4-{2-[4-(5-Cyano-1H-indol-3-yl)-butylamino]-ethoxy}-benzofuran-2-carboxylic acid methyl ester |
| 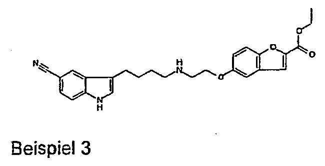 | 445,5 | | trifluoroacetate | 559,5 | 5-{2-[4-(5-Cyano-1H-indol-3-yl)-butylamino]-ethoxy}-benzofuran-2-carboxylic acid ethyl ester |
| 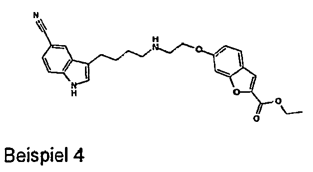 | 445,5 | | trifluoroacetate | 559,5 | 6-{2-[4-(5-Cyano-1H-indol-3-yl)-butylamino]-ethoxy}-benzofuran-2-carboxylic acid ethyl ester |
| 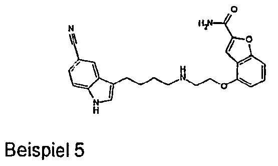 | 416,5 | | bis-trifluoroacetate | 644,5 | 4-{2-[4-(5-Cyano-1H-indol-3-yl)-butylamino]-ethoxy}-benzofuran-2-carboxylic acid amide |

| **Beispiele** | **MW** | **SALZ** | **MW-SALZ** | **NAME** |
|---|---|---|---|---|
| 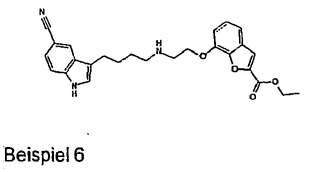 | 445,5 | trifluoroacetate | 559,5 | 7-{2-[4-(5-Cyano-1H-indol-3-yl)-butylamino]-ethoxy}-benzofuran-2-carboxylic acid ethyl ester |
| 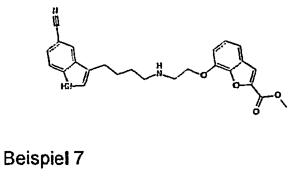 | 431,5 | | | 7-{2-[4-(5-Cyano-1H-indol-3-yl)-butylamino]-ethoxy}-benzofuran-2-carboxylic acid methyl ester |
| 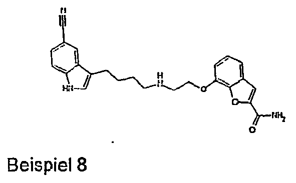 | 416,5 | | | 7-{2-[4-(5-Cyano-1H-indol-3-yl)-butylamino]-ethoxy}-benzofuran-2-carboxylic acid amide |
| 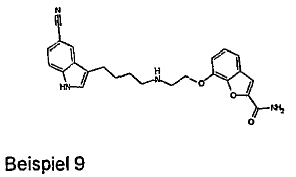 | 416,5 | bis-trifluoroacetate | 644,5 | 7-{2-[4-(5-Cyano-1H-indol-3-yl)-butylamino]-ethoxy}-benzofuran-2-carboxylic acid amide |
| 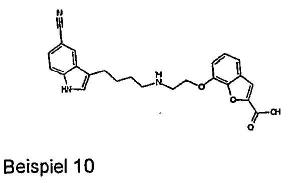 | 417,5 | | | 7-{2-[4-(5-Cyano-1H-indol-3-yl)-butylamino]-ethoxy}-benzofuran-2-carboxylic acid |
| 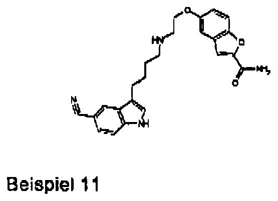 | 416,5 | | | 5-{2-[4-(5-Cyano-1H-indol-3-yl)-butylamino]-ethoxy}-benzofuran-2-carboxylic acid amide |
| 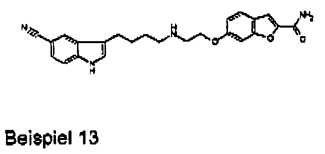 | 416,5 | | | 6-{2-[4-(5-Cyano-1H-6-{2-[4-(5-Cyano-1H-indol-3-yl)-butylamino]-ethoxy}-benzofuran-2-carboxylic acid amide |
| 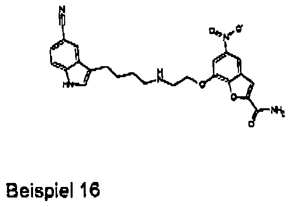 | 461,5 | | | 7-{2-[4-(5-Cyano-1H-indol-3-yl)-butylamino]-ethoxy}-5-nitro-benzofuran-2-carboxylic acid amide |
| 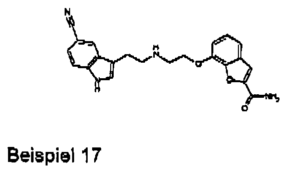 | 388,4 | bis-trifluoroacetate | 616,5 | 7-{2-[2-(5-Cyano-1H-indol-3-yl)-ethylamino]-ethoxy}-benzofuran-2-carboxylic acid amide |
| 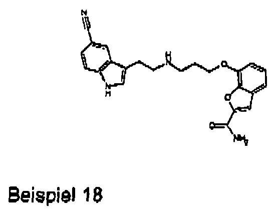 | 402,5 | | | 7-{3-[2-(5-Cyano-1H-indol-3-yl)-ethylamino]-propoxy}-benzofuran-2-carboxylic acid amide |

### Charakterisierung der o.g. Beispiele:

| **Beispiel** | **MW** | **MS-Ergebnis** |
|---|---|---|
| 2 | 431,5 | MALDI-MS (M+H)⁺ 432 |
| 3 | 445.5 | MALDI-MS (M+H)⁺ 446 |
| 4 | 445.5 | EI-MS (M)⁺ 445 |
| 5 | 416.5 | HPLC-ESI-MS (M + H)⁺ 417 |
| 6 | 445.5 | HPLC-ESI-MS (M + H)⁺ 446 |
| 7 | 431.5 | HPLC-ESI-MS (M + H)⁺ 432 |
| 8 | 416.5 | EI-MS (M)⁺ 416 |
| 9 | 416.5 | EI-MS (M)⁺ 416 |
| 10 | 417.5 | HPLC-ESI-MS (M + H)⁺ 418 |
| 11 | 416.5 | HPLC-ESI-MS (M + H)⁺ 417 |
| 13 | 416.5 | HPLC-ESI-MS (M + H)⁺ 417 |
| 16 | 461.5 | EI-MS (M)⁺ 461 |
| 17 | 388.4 | HPLC-MS (M + H)⁺ 389 |
| 18 | 402.5 | HPLC-ESI-MS (M + H)⁺ 403 |

| | | |
|---|---|---|
| EI-MS: Electon Impact Massen-Spektroskopie ESI-MS: Elektrospray Massen-Spektroskopie MALDI-MS: Matrix Assisted Laser Desorption/Ionization Massen-Spektroskopie | | |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepresst, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so dass jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 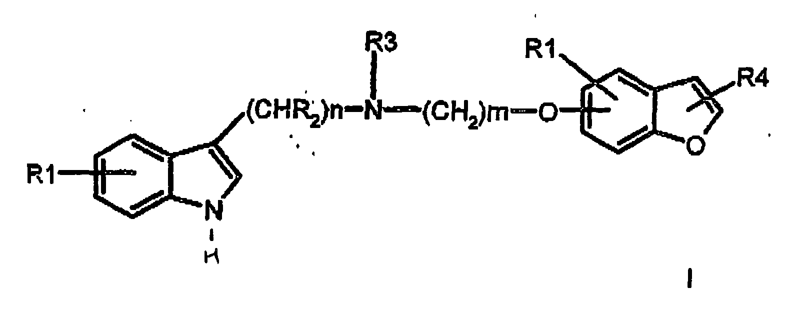 worin
R¹ H, Ein- oder Zweifachsubstitution durch CN,
R OH, OA, NH₂, NHA oder NA₂
R², R³ H oder A
R⁴ Ein- oder Zweifachsubstitution durch COR
A Alkyl mit 1, 2, 3, 4, 5 oder 6 Atomen
Hal F, Cl, Br oder I
m 2, 3, 4, 5 oder 8
n 1, 2, 3 oder 4
bedeuten,
sowie ihre Solvate und Stereoisomere, einschließlich deren Mischungen in allen Verhältnissen.

2. Verbindungen nach Anspruch 1, worin
R¹ CN
R NH2, OH, O-Methyl oder O-Ethyl
bedeuten,
sowie ihre Solvate und Stereoisomere, einschließlich deren Mischungen in allen Verhältnissen.

3. Verbindungen nach Anspruch 1 oder 2, worin
R² H oder Methyl
R³ H oder Methyl
bedeuten,
sowie ihre Solvate und Stereoisomere, einschließlich deren Mischungen in allen Verhältnissen.

4. Verbindungen nach einem oder mehreren der Ansprüche 1 bis 3,
worin
A Methyl oder Ethyl
bedeuten,
sowie ihre Solvate und Stereoisomere, einschließlich deren Mischungen in allen Verhältnissen.

5. Verbindungen nach einem oder mehreren der Ansprüche 1 bis 4,
worin
R⁴ CONH₂, COOC₂H₅ oder CONHA
bedeuten,
sowie ihre Solvate und Stereoisomere, einschließlich deren Mischungen in allen Verhältnissen.

6. Verbindungen nach einem oder mehreren der Ansprüche 1 bis 5,
worin
m 2 oder 3
n 2 oder 4
bedeuten,
sowie ihre Solvate und Stereoisomere, einschließlich deren Mischungen in allen Verhältnissen.

7. Verbindungen nach Anspruch 1
4-{2-[4-(5-Cyano-1H-indol-3-yl)-butylamino]-ethoxy}-benzofuran-2-carboxylic acid methyl ester,
5-{2-[4-(5-Cyano-1H-indol-3-yl)-butylamino]-ethoxy}-benzofuran-2-carboxylic acid ethyl ester,
6-{2-[4-(5-Cyano-1H-indol-3-yl)-butylamino]-ethoxy}-benzofuran-2-carboxylic acid ethyl ester,
4-{2-[4-(5-Cyano-1H-indol-3-yl)-butylamino]-ethoxy}-benzofuran-2-carboxylic acid amide,
7-{2-[4-(5-Cyano-1H-indol-3-yl)-butylamino]-ethoxy}-benzofuran-2-carboxylic acid ethyl ester,
7-{2-[4-(5-Cyano-1H-indol-3-yl)-butylamino]-ethoxy}-benzofuran-2-carboxylic acid methyl ester,
7-{2-[4-(5-Cyano-1H-indol-3-yl)-butylamino]-ethoxy}-benzofuran-2-carboxylic acid amide,
7-{2-[4-(5-Cyano-1H-indol-3-yl)-butylamino]-ethoxy}-benzofuran-2-carboxylic acid,
5-{2-[4-(5-Cyano-1H-indol-3-yl)-butylamino]-ethoxy}-benzofuran-2-carboxylic acid amide,
6-{2-[4-(5-Cyano-1H-indol-3-yl)-butylamino]-ethoxy}-benzofuran-2-carboxylic acid amide,
7-{2-[4-(5-Cyano-1H-indol-3-yl)-butylamino]-ethoxy}-5-nitro-benzofuran-2-carboxylic acid amide,
7-{2-[2-(5-Cyano-1H-indol-3-yl)-ethylamino]-ethoxy}-benzofuran-2-carboxylic acid amide,
7-{3-[2-(5-Cyano-1H-indol-3-yl)-ethylamino]-propoxy}-benzofuran-2-carboxylic acid amide,

8. Verfahren zur Herstellung von Verbindungen der Formel I nach den Ansprüchen 1 bis 7 sowie ihrer Solvate und Stereoisomere, **dadurch gekennzeichnet, dass** man
eine Verbindung der Formel II 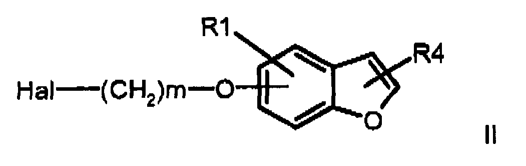 worin
R¹, R⁴, Hal und m die in Anspruch 1 angegebenen Bedeutungen haben
mit einer Verbindung der Formel III 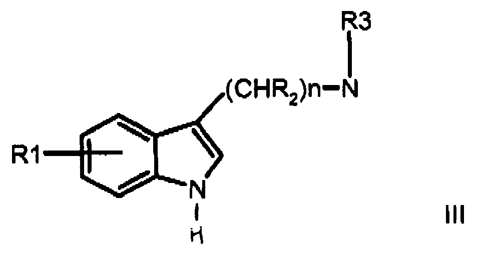 worin
R¹, R² R³ und n die in Anspruch 1 angegebenen Bedeutungen haben,
umsetzt und/oder
eine basische oder saure Verbindung der Formel I durch Behandeln mit einer Säure oder Base in eines ihrer Salze oder Solvate umwandelt.

9. Verbindungen der Formel I nach einem oder mehreren der Ansprüche 1 bis 7 und ihre physiologisch unbedenklichen Salze oder Solvate als 5 HT_{1A} -Agonisten, 5HT_{1D} und/oder 5 HT_{2A} und als Inhibitoren der 5-HT-Wiederaufnahme.

10. Arzneimittel, enthaltend mindestens eine Verbindung der Formel I nach einem oder mehreren Ansprüche 1 bis 7 und/oder ihre Solvate und Stereoisomere, einschließlich deren Mischungen in allen Verhältnissen, sowie gegebenenfalls Träger- und/oder Hilfsstoffe.

11. Arzneimittel, enthaltend mindestens eine Verbindung der Formel I nach einem oder mehreren der Ansprüche 1 bis 7 und/oder ihre Solvate und Stereoisomere, einschließlich deren Mischungen in allen Verhältnissen, und mindestens einen weiteren Arzneimittelwirkstoff.

12. Verwendung von Verbindungen der Formel I nach einem oder mehreren der Ansprüche 1 bis 7 und/oder ihrer Solvate und Stereoisomere, einschließlich deren Mischungen in allen Verhältnissen, zur Herstellung eines Arzneimittels.

13. Verwendung von Verbindungen der Formel I nach einem oder mehreren der Ansprüche 1 bis 7 und/oder ihrer Solvate und Stereoisomere, einschließlich deren Mischungen in allen Verhältnissen, zur Herstellung eines Arzneimittels zur Bekämpfung von Krankheiten, die mit dem Serotonin-Neurotransmittersystem verbunden sind und bei denen hochaffine Serotoninrezeptoren (5-HT_{1A}-Rezeptoren) und/oder 5HT_{1D}- Rezeptoren beteiligt sind.

14. Verwendung von Verbindungen der Formel I nach einem oder mehreren der Ansprüche 1 bis 7 und /oder ihrer Solvate und Stereoisomere, einschließlich deren Mischungen in allen Verhältnissen, zur Herstellung eines Arzneimittels als Anxiolytikum, Antidrepressivum, Neuroleptikum und/oder Antihypertonikum.

15. Verwendung von Verbindungen der Formel I nach einem oder mehreren der Ansprüche 1 bis 7 und/oder ihrer Solvate und Stereoisomere, einschließlich deren Mischungen in allen Verhältnissen, zur Herstellung eines Arzneimittels zur Bekämpfung von Psychosen, der Symptome von Morbus-Alzheimer, krankhaften Angstzuständen, Übererregung, Hyperaktivität, Aufmerksamkeitsstörungen bei Kindern und Jugendlichen, tiefgreifende Entwicklungsstörungen und Störungen des Sozialverhaltens mit geistiger Retardierung, Depression, Zwangserkrankungen im engeren (OCD) und weiteren Sinne (OCSD), sexuelle Funktionsstörungen, Schlafstörungen, Störungen in der Nahrungsaufnahme, sowie solcher psychiatrischer Symptome in Rahmen der Altersdemenz und der Demenz vom Alzheimer-Typ, zur Verminderung von kognitiven Leistungsstörungen oder zur Prophylaxe und Kontrolle von Himinfakten (Apoplexia cerebri), zur Behandlung von extrapyramidalen Bewegungserkrankungen, zur Behandlung von Nebenwirkungen, die bei der Behandlung von extrapyramidalen Bewegungserkrankungen mit konventionellen Anti-Parkinson-Medikamenten auftreten oder zur Behandlung von extrapyramidalen Symptomen (EPS), die durch Neuroleptika induziert werden.

16. Set (Kit), bestehend aus getrennten Packungen von
(a) einer wirksamen Menge an einer Verbindung der Formel I nach einem oder mehreren der Ansprüche 1 bis 7 und/oder ihrer Solvate und Stereoisomere, einschließlich deren Mischungen in allen Verhältnissen, und
(b) einer wirksamen Menge eines weiteren Arzneimittelwirkstoffs.

## Claims

1. Compounds of the formula I 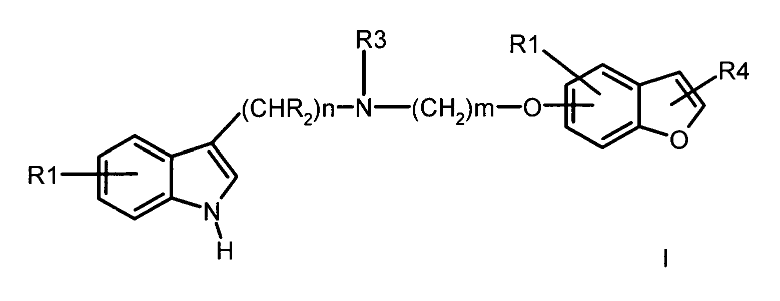 in which
R¹ denotes H, mono- or disubstitution by CN,
R denotes OH, OA, NH₂, NHA or NA₂
R², R³ denote H or A
R⁴ denotes mono- or disubstitution by COR
A denotes alkyl having 1, 2, 3, 4, 5 or 6 atoms
Hal denotes F, Cl, Br or I
m denotes 2, 3, 4, 5 or 6
n denotes 1, 2, 3 or 4,
and solvates and stereoisomers thereof, including mixtures thereof in all ratios.

2. Compounds according to Claim 1 in which
R¹ denotes CN
R denotes NH₂, OH, O-methyl or O-ethyl,
and solvates and stereoisomers thereof, including mixtures thereof in all ratios.

3. Compounds according to Claim 1 or 2 in which
R² denotes H or methyl
R³ denotes H or methyl,
and solvates and stereoisomers thereof, including mixtures thereof in all ratios.

4. Compounds according to one or more of Claims 1 to 3 in which
A denotes methyl or ethyl,
and solvates and stereoisomers thereof, including mixtures thereof in all ratios.

5. Compounds according to one or more of Claims 1 to 4 in which
R⁴ denotes CONH₂, COOC₂H₅ or CONHA,
and solvates and stereoisomers thereof, including mixtures thereof in all ratios.

6. Compounds according to one or more of Claims 1 to 5 in which
m denotes 2 or 3
n denotes 2 or 4,
and solvates and stereoisomers thereof, including mixtures thereof in all ratios.

7. Compounds according to Claim 1
4-{2-[4-(5-cyano-1H-indol-3-yl)butylamino]ethoxy}benzofuran-2-carboxylic acid methyl ester,
5-{2-[4-(5-cyano-1H-indol-3-yl)butylamino]ethoxy}benzofuran-2-carboxylic acid ethyl ester,
6-{2-[4-(5-cyano-1H-indol-3-yl)butylamino]ethoxy}benzofuran-2-carboxylic acid ethyl ester,
4-{2-[4-(5-cyano-1H-indol-3-yl)butylamino]ethoxy}benzofuran-2-carboxylic acid amide,
7-{2-[4-(5-cyano-1H-indol-3-yl)butylamino]ethoxy}benzofuran-2-carboxylic acid ethyl ester,
7-{2-[4-(5-cyano-1H-indol-3-yl)butylamino]ethoxy}benzofuran-2-carboxylic acid methyl ester,
7-{2-[4-(5-cyano-1H-indol-3-yl)butylamino]ethoxy}benzofuran-2-carboxylic acid amide,
7-{2-[4-(5-cyano-1H-indol-3-yl)butylamino]ethoxy}benzofuran-2-carboxylic acid,
5-{2-[4-(5-cyano-1H-indol-3-yl)butylamino]ethoxy}benzofuran-2-carboxylic acid amide,
6-{2-[4-(5-cyano-1H-indol-3-yl)butylamino]ethoxy}benzofuran-2-carboxylic acid amide,
7-{2-[4-(5-cyano-1H-indol-3-yl)butylamino]ethoxy}-5-nitrobenzofuran-2-carboxylic acid amide,
7-{2-[2-(5-cyano-1H-indol-3-yl)ethylamino]ethoxy}benzofuran-2-carboxylic acid amide,
7-{3-[2-(5-cyano-1H-indol-3-yl)ethylamino]propoxy}benzofuran-2-carboxylic acid amide.

8. Process for the preparation of compounds of the formula I according to Claims 1 to 7 and solvates and stereoisomers thereof, **characterised in that**
a compound of the formula II 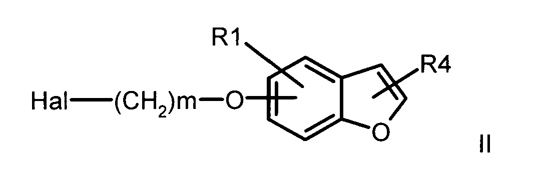 in which
R¹, R⁴, Hal and m have the meanings indicated in Claim 1
is reacted with a compound of the formula III 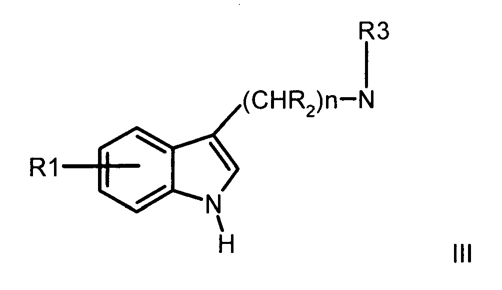 in which
R¹, R² R³ and n have the meanings indicated in Claim 1,
and/or
a basic or acidic compound of the formula I is converted into one of its salts or solvates by treatment with an acid or base.

9. Compounds of the formula I according to one or more of Claims 1 to 7 and physiologically acceptable salts or solvates thereof as 5 HT_{1A}, 5HT_{1D} and/or 5 HT_{2A} agonists and as 5-HT reuptake inhibitors.

10. Medicaments comprising at least one compound of the formula I according to one or more Claims 1 to 7 and/or solvates and stereoisomers thereof, including mixtures thereof in all ratios, and optionally excipients and/or adjuvants.

11. Medicaments comprising at least one compound of the formula I according to one or more of Claims 1 to 7 and/or solvates and stereoisomers thereof, including mixtures thereof in all ratios, and at least one further medicament active ingredient.

12. Use of compounds of the formula I according to one or more of Claims 1 to 7 and/or solvates and stereoisomers thereof, including mixtures thereof in all ratios, for the preparation of a medicament.

13. Use of compounds of the formula I according to one or more of Claims 1 to 7 and/or solvates and stereoisomers thereof, including mixtures thereof in all ratios, for the preparation of a medicament for combating diseases which are associated with the serotonin neurotransmitter system and in which high-affinity serotonin receptors (5-HT_{1A} receptors) and/or 5HT_{1D} receptors are involved.

14. Use of compounds of the formula I according to one or more of Claims 1 to 7 and/or solvates and stereoisomers thereof, including mixtures thereof in all ratios, for the preparation of a medicament as anxiolytic, antidepressant, neuroleptic and/or antihypertonic.

15. Use of compounds of the formula I according to one or more of Claims 1 to 7 and/or solvates and stereoisomers thereof, including mixtures thereof in all ratios, for the preparation of a medicament for combating psychoses, the symptoms of Alzheimer's disease, pathological anxiety states, overexcitation, hyperactivity, attention disorders in children and youths, severe developmental disorders and disorders of social behaviour with mental retardation, depression, obsessive-compulsive disorders in the narrower (OCD) and broader sense (OCSD), sexual dysfunctions, sleeping disorders, disorders in nutrient uptake, and psychiatric symptoms as part of age dementia and dementia of the Alzheimer's type, for reducing defects in cognitive ability, or for the prophylaxis and control of cerebral infarctions (apoplexia cerebri), for the treatment of extrapyramidal motor diseases, for the treatment of side effects which occur in the treatment of extrapyramidal motor diseases with conventional anti-Parkinson's medicaments, or for the treatment of extrapyramidal symptoms (EPS) induced by neuroleptics.

16. Set (kit) consisting of separate packs of
(a) an effective amount of a compound of the formula I according to one or more of Claims 1 to 7 and/or solvates and stereoisomers thereof, including mixtures thereof in all ratios, and
(b) an effective amount of a further medicament active ingredient.

## Revendications

1. Composés de formule I 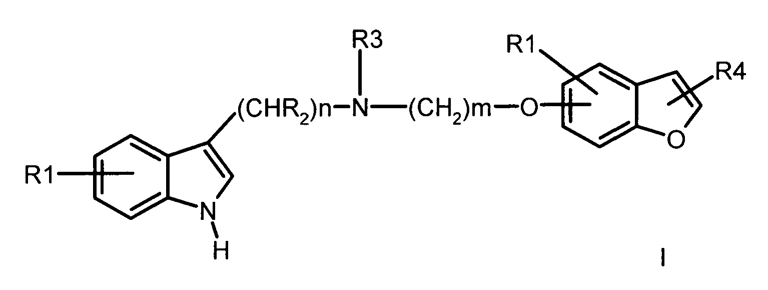 dans laquelle
R¹ désigne H, une mono- ou disubstitution par CN,
R désigne OH, OA, NH₂, NHA ou NA₂
R², R³ désignent H ou A
R⁴ désigne une mono- ou disubstitution par COR
A désigne alkyle ayant 1, 2, 3, 4, 5 ou 6 atomes
Hal désigne F, Cl, Br ou I
m désigne 2, 3, 4, 5 ou 6
n désigne 1, 2, 3 ou 4,
et les solvats et stéréoisomères de ceux-ci, y compris des mélanges de ceux-ci en toutes proportions.

2. Composés selon la revendication 1, dans lesquels
R¹ désigne CN
R désigne NH₂, OH, O-méthyle ou O-éthyle,
et les solvats et stéréoisomères de ceux-ci, y compris des mélanges de ceux-ci en toutes proportions.

3. Composés selon la revendication 1 ou 2, dans lesquels
R² désigne H ou méthyle,
R³ désigne H ou méthyle,
et les solvats et stéréoisomères de ceux-ci, y compris des mélanges de ceux-ci en toutes proportions.

4. Composés selon l'une ou plusieurs parmi les revendications 1 à 3, dans lesquels
A désigne méthyle ou éthyle,
et les solvats et stéréoisomères de ceux-ci, y compris des mélanges de ceux-ci en toutes proportions.

5. Composés selon l'une ou plusieurs parmi les revendications 1 à 4, dans lesquels
R⁴ désigne CONH₂, COOC₂H₅ ou CONHA,
et les solvats et stéréoisomères de ceux-ci, y compris des mélanges de ceux-ci en toutes proportions.

6. Composés selon l'une ou plusieurs parmi les revendications 1 à 5, dans lesquels
m désigne 2 ou 3
n désigne 2 ou 4,
et les solvats et stéréoisomères de ceux-ci, y compris des mélanges de ceux-ci en toutes proportions.

7. Composés selon la revendication 1
l'ester méthylique de l'acide 4-{2-[4-(5-cyano-1H-indol-3-yl)butylamino]éthoxy}benzofuran-2-carboxylique,
l'ester éthylique de l'acide 5-{2-[4-(5-cyano-1H-indol-3-yl)butylamino]-éthoxy}benzofuran-2-carboxylique,
l'ester éthylique de l'acide 6-{2-[4-(5-cyano-1H-indol-3-yl)butylamino]-éthoxy}benzofuran-2-carboxylique,
l'acide 4-{2-[4-(5-cyano-1H-indol-3-yl)butylamino]éthoxy}benzofuran-2-carboxylique amide,
l'ester éthylique de l'acide 7-{2-[4-(5-cyano-1H-indol-3-yl)butylamino]-éthoxy}benzofuran-2-carboxylique,
l'ester méthylique de l'acide 7-{2-[4-(5-cyano-1H-indol-3-yl)butylamino]éthoxy}benzofuran-2-carboxylique,
l'acide 7-{2-[4-(5-cyano-1H-indol-3-yl)butylamino]éthoxy}benzofuran-2-carboxylique amide,
l'acide 7-{2-[4-(5-cyano-1H-indol-3-yl)butylamino]éthoxy}benzofuran-2-carboxylique,
l'acide 5-{2-[4-(5-cyano-1H-indol-3-yl)butylamino]éthoxy}benzofuran-2-carboxylique amide,
l'acide 6-{2-[4-(5-cyano-1H-indol-3-yl)butylamino]éthoxy}benzofuran-2-carboxylique amide,
l'acide 7-{2-[4-(5-cyano-1H-indol-3-yl)butylamino]éthoxy}-5-nitro-benzofuran-2-carboxylique amide,
l'acide 7-{2-[2-(5-cyano-1H-indol-3-yl)éthylamino]éthoxy}benzofuran-2-carboxylique amide,
l'acide 7-{3-[2-(5-cyano-1H-indol-3-yl)éthylamino]propoxy}benzofuran-2-carboxylique amide.

8. Procédé de préparation de composés de formule I selon les revendications 1 à 7, et de solvats et stéréoisomères de ceux-ci, **caractérisé en ce que**
un composé de formule II 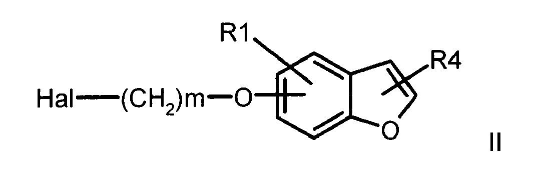 dans laquelle
R¹, R⁴, Hal et m ont les significations indiquées selon la revendication 1
est réagi avec un composé de formule III 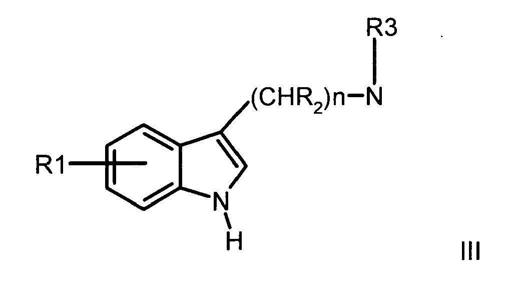 dans laquelle
R¹, R² R³ et n ont les significations indiquées selon la revendication 1,
et/ou
un composé basique ou acide de formule I est converti en l'un de ses sels ou solvats par traitement par un acide ou une base.

9. Composés de formule I selon l'une ou plusieurs parmi les revendications 1 à 7, et sels ou solvats physiologiquement acceptables de ceux-ci comme agonistes de la 5 HT_{1A}, 5HT_{1D} et/ou 5 HT_{2A} et comme inhibiteurs du recaptage de la 5-HT.

10. Médicaments comprenant au moins un composé de formule I selon l'une ou plusieurs parmi les revendications 1 à 7, et/ou des solvats et stéréoisomères de ceux-ci, y compris des mélanges de ceux-ci en toutes proportions, et éventuellement des excipients et/ou adjuvants.

11. Médicaments comprenant au moins un composé de formule I selon l'une ou plusieurs parmi les revendications 1 à 7, et/ou des solvats et stéréoisomères de ceux-ci, y compris des mélanges de ceux-ci en toutes proportions, et au moins un ingrédient actif médicamenteux supplémentaire.

12. Utilisation de composés de formule I selon l'une ou plusieurs parmi les revendications 1 à 7, et/ou de solvats et stéréoisomères de ceux-ci, y compris des mélanges de ceux-ci en toutes proportions, pour la préparation d'un médicament.

13. Utilisation de composés de formule I selon l'une ou plusieurs parmi les revendications 1 à 7, et/ou de solvats et stéréoisomères de ceux-ci, y compris des mélanges de ceux-ci en toutes proportions, pour la préparation d'un médicament destiné à lutter contre des maladies liées au système neurotransmetteur de la sérotonine et dans lequel des récepteurs de sérotonine à haute affinité (récepteurs 5-HT_{1A}) et/ou des récepteurs 5HT_{1D} sont impliqués.

14. Utilisation de composés de formule I selon l'une ou plusieurs parmi les revendications 1 à 7, et/ou de solvats et stéréoisomères de ceux-ci, y compris des mélanges de ceux-ci en toutes proportions, pour la préparation d'un médicament comme agent anxiolytique, antidépresseur, neuroleptique et/ou antihypertonique.

15. Utilisation de composés de formule I selon l'une ou plusieurs parmi les revendications 1 à 7, et/ou de solvats et stéréoisomères de ceux-ci, y compris des mélanges de ceux-ci en toutes proportions, pour la préparation d'un médicament destiné à lutter contre les psychoses, les symptômes de la maladie d'Alzheimer, les états d'anxiété pathologique, la surexcitation, l'hyperactivité, les troubles de l'attention chez les enfants et les adolescents, les troubles de développement sévères et les troubles de comportement social avec déficience mentale, la dépression, les troubles obsessionnels-compulsifs au sens restreint (OCD) et large (OCSD), les dysfonctionnements sexuels, les troubles du sommeil, les troubles de l'alimentation, et les symptômes psychiatriques faisant partie d'une démence liée à l'âge et d'une démence de type Alzheimer, pour la réduction des défauts de la capacité cognitive, ou pour la prophylaxie et le contrôle des infarctus cérébraux (apoplexia cerebri), pour le traitement de maladies du système moteur extrapyramidal, pour le traitement des effets secondaires survenant lors du traitement des maladies du système moteur extrapyramidal par des médicaments antiparkinsoniens classiques, ou pour le traitement des symptômes extrapyramidaux (EPS) induits par des neuroleptiques.

16. Ensemble (kit) constitué de conditionnements distincts
(a) d'une quantité efficace d'un composé de formule I selon l'une ou plusieurs parmi les revendications 1 à 7, et/ou de solvats et stéréoisomères de ceux-ci, y compris des mélanges de ceux-ci en toutes proportions, et
(b) d'une quantité efficace d'un autre ingrédient actif médicamenteux.
